# EUROPÄISCHE PATENTSCHRIFT

(11) **EP 0 497 852 B1**
(45) Veröffentlichungstag und Bekanntmachung des Hinweises auf die Patenterteilung: **20.12.1995**
(21) Anmeldenummer: 90916287.7
(22) Anmeldetag: 20.10.1990
(51) Int. Cl.: C07D 403/04, A61K 31/415, C07D 401/14, C07D 209/34

(54) **BICYCLO-IMIDAZOLE, VERFAHREN ZU IHRER HERSTELLUNG UND DIESE VERBINDUNGEN ENTHALTENDE ARZNEIMITTEL**
BICYCLO-IMIDAZOLES, PROCESS FOR PRODUCING THEM AND DRUGS CONTAINING THEM
BICYCLO-IMIDAZOLES, PROCEDE POUR LEUR FABRICATION ET MEDICAMENTS LES CONTENANT

(30) Priorität: 25.10.1989 DE 3935514
(43) Veröffentlichungstag der Anmeldung: 12.08.1992
(73) Patentinhaber: BOEHRINGER MANNHEIM GMBH, 68298 Mannheim (DE)
(72) Erfinder: VON DER SAAL, Wolfgang, D-6940 Weinheim (DE); MERTENS, Alfred, D-6905 Schriesheim (DE); BÖHM, Erwin, D-6802 Ladenburg (DE); KLING, Lothar, D-6800 Mannheim 1 (DE)
(74) Vertreter: Weber, Manfred, Dr.
(86) Internationale Anmeldenummer: EP9001788
(87) Internationale Veröffentlichungsnummer: WO9106545

(56) Entgegenhaltungen:
- EP-A- 0 148 623
- EP-A- 0 211 698
- EP-A- 0 257 918
- EP-A- 0 344 634
- WO-A-86/03749
- PATENT ABSTRACTS OF JAPAN, Band 6, Nr. 191 (C-127)(1069), 30. September 1982, & JP, A, 57102863
- PATENT ABSTRACTS OF JAPAN, Band 11, Nr. 242 (C-438)(2689), 7. August 1987, & JP, A, 6248679

## Beschreibung

Die vorliegende Erfindung betrifft neue Verbindungen der allgemeinen Formel I 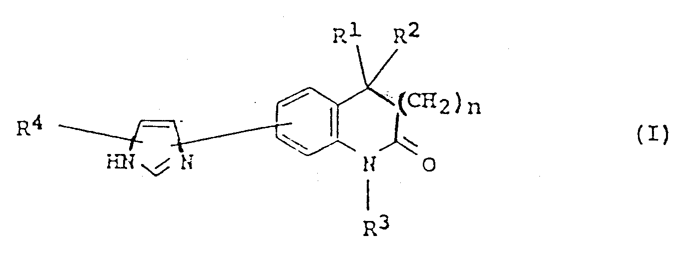
in welcher
- R¹: ein Wasserstoffatom, eine C₁-C₆-Alkyl-, C₂-C₆-Alkenyl- oder eine C₃-C₇-Cycloalkylgruppe bedeutet,
- R²: eine C₁-C₆-Alkylgruppe bedeutet oder R¹ und R² zusammen mit den Kohlenstoffatom, an das sie gebunden sind, einen C₃-C₇-Spirocyclus bilden,
- n: gleich 0 oder 1 sein kann,
- R³: ein Wasserstoffatom oder eine C₁-C₈-Alkylgruppe bedeutet,
- R⁴: einen in 2- oder 4-Stellung des Imidazols befindlichen Pyridinylring oder einen Phenylring der allgemeinen Formel II bedeutet,

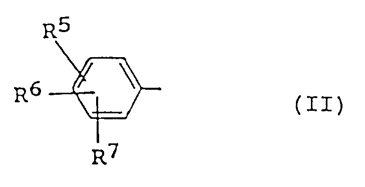

in dem
R⁵, R⁶, R⁷ gleich oder verschieden sein können und jeweils Wasserstoff, Halogen oder C₁-C₇-Alkoxy bedeuten,
wobei im Fall n=0 der Imidazolring in 4-, 5-, 6- oder 7-Stellung mit dem 2,3-Dihydroindolin-2-on oder im Fall n=1 in 5-, 6-, 7-, 8-Stellung mit dem 1,2,3,4-Tetrahydrochinolin-2-on verknüpft sein kann, deren Tautomere optisch aktive Formen oder deren physiologisch verträgliche Salze.

Unter Tautomeren der allgemeinen Formel I ist die Tautomerie des Imidazols von besonderer Bedeutung. Befindet sich der Rest R⁴ in 2-Stellung des Imidazols (allgemeine Formel I'), so kann der Bicyclus über 4- oder 5-Stellung des Imidazolrings gebunden sein: 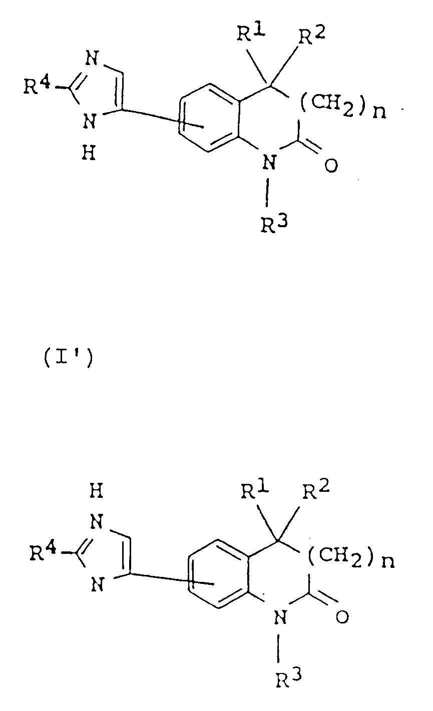
In dieser Patentanmeldung wird im weiteren nur eine der tautomeren Formen von I' benutzt, wobei damit auch die andere tautomere Form gemeint ist. Befindet sich der Bicyclus in 2-Stellung des Imidazols, (allgemeine Formel I''), so kann der Rest R⁴ in 4- oder 5-Stellung des Imidazols vorhanden sein: 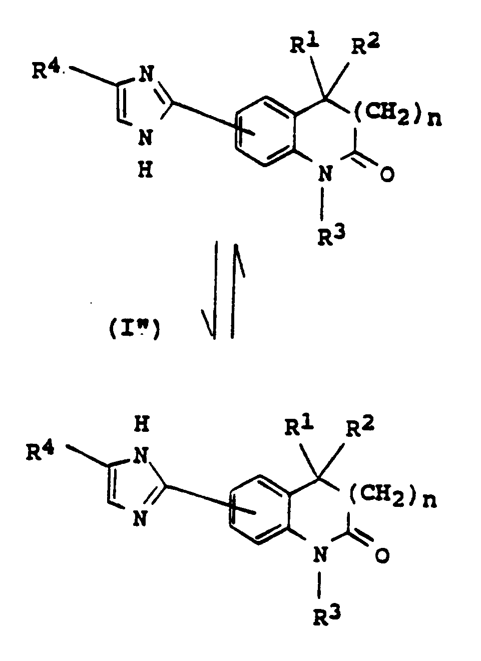
In dieser Patentanmeldung wird im weiteren nur die eine der tautomeren Formen von I'' benutzt, wobei damit auch die andere tautomere Form gemeint ist.

Es wurde nun überraschend gefunden, daß die Verbindungen der allgemeinen Formel I sowohl die Erythrozytenaggregation als auch die Thrombozytenaggregation in geringen Konzentrationen hemmen, und sich damit als therapeutisch aktive Wirkstoffe zur Herstellung von Arzneimitteln eignen.

Aufgrund dieser Eigenschaften sind diese Substanzen geeignet zur Behandlung von Krankheiten, bei denen in der Pathogenese die Erythrozyten- und Thrombozytenaggregation eine wichtige Rolle spielen, wie zum Beispiel periphere, coronare und cerebrale Durchblutungsstörungen, Schockzustände, degenerative Gefäßerkrankungen, rheumatische Erkrankungen, verschiedene Arten von Ulcera, nekrotischen Prozessen in Tumoren, degenerative Störungen der Retina, Nerven und Muskeln oder von verschiedenen Hautkrankheiten. Insbesondere kommt die Behandlung von arteriellen Verschlußkrankheiten, ischämischen Zuständen, venöser Insuffizienz oder Diabetes mellitus in Frage. Die erfindungsgemäßen Verbindungen besitzen eine überraschend lang anhaltende Wirkung. Sie können vorteilhaft durch orale Gabe appliziert werden.

Verbindungen ähnlicher Struktur wie die der allgemeinen Formel I sind bekannt. So beschreibt die Japanische Patentanmeldung JP 62/48679 (Otsuka) 6-Heterocyclylcarbostyrile mit cardiotoner Wirkung, und darunter spezifisch die Verbindungen III. 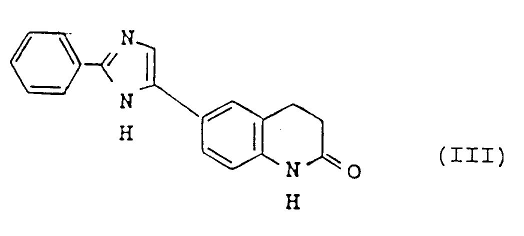
Diese Verbindungen der Formel III unterscheiden sich von den erfindungsgemäßen Verbindungen dadurch, daß die beiden Substituenten R¹ und R² in Formel I nicht gleichzeitig ein Wasserstoffatom bedeuten können.

Strukturell den Verbindungen der Formel I nahe Verbindungen werden auch in EP-A-0 344 634 beschrieben. Diese Verbindungen enthalten jedoch im Gegensatz zu den erfindungsgemäßen anstelle der Imidazolylseitenkette eine Carboxamidseitenkette.

Die Alkoxygruppe für R⁵, R⁶ und R⁷ kann 1-7 Kohlenstoffatome enthalten, vorzugsweise 1-4 Kohlenstoffatome, und geradkettig oder verzweigt sein. Bevorzugt in diesem Sinne sind beispielsweise die Methoxy-, Ethoxy- und Propyloxygruppe.

Insbesondere sind bevorzugt
für R⁵ Wasserstoff oder ein Halogenatom,
für R⁶ Wasserstoff oder ein Halogenatom und
für R⁷ Wasserstoff oder die Methoxygruppe.

Die Substituenten R⁵, R⁶, R⁷ können unabhängig voneinander in 2-, 3- oder 4-Stellung des Phenylringes II stehen, wobei der Phenylring 1 bis 3 der genannten Substituenten tragen kann.

Bevorzugte monosubstituierte Phenyle sind die C₁-C₇-Alkoxy- oder Halogenphenyle, wobei der Substituent vorzugsweise in 4- oder 5-Stellung stehen kann.

Bevorzugte disubstituierte Phenyle enthalten als Substituenten Alkoxy und Halogen, wobei die beiden Substituenten gleich oder verschieden sein können und die vorgenannten Alkylreste, allein oder in Kombination mit anderen Resten, vorzugsweise 1-3 C-Atome aufweisen können.

Für den Fall, daß R⁵ in 2-Stellung (ortho) am Phenylring steht, so bedeutet R⁵ vorzugsweise ein Wasserstoffatom. Bezeichnet man R⁶ als den in 3-Stellung (meta) befindlichen Substituenten, so kommen insbesondere ein Wasserstoff- oder ein Halogenatom, wie z. B. Chloratom in Frage. Ist R⁷ der in 4-Stellung (para) befindliche Substituent, dann bedeutet R⁷ insbesondere ein Wasserstoffatom, eine C₁-C₆-Alkoxygruppe, wie z. B. Methoxy, Ethoxy, Isopropyloxy, oder ein Halogenatom, wie z. B. ein Fluor- oder Chloratom.

Bedeutet R¹ eine Alkyl-, Alkenyl- oder Cycloalkylgruppe und R² eine Alkylgruppe, so kann jeder der vorgenannten Alkyl- oder Alkenylteile geradkettig oder verzweigt sein und 1-6 bzw. 2-6 Kohlenstoffatome und der genannte Cycloalkylteil 3-7 Kohlenstoffatome enthalten.

Bevorzugt in diesem Sinne ist für R¹ ein Wasserstoffatom, die Methyl-, Ethyl-, Isopropyl-, 3-Pentyl-, Cyclopentyl- oder Cyclohexylgruppe. R² kann vorzugsweise eine Methyl-, Ethyl-, Isopropyl - oder 3-Pentylgruppe darstellen.

Bilden R¹ und R² zusammen mit dem C-Atom, an das sie gebunden sind, einen Cycloalkylring, so handelt es sich dabei vorzugsweise um die Spirocyclopropyl-, Spirocyclobutyl-, Spirocyclopentyl- und Spirocyclohexylgruppe.

Besonders bevorzugte Reste für R₃ sind das Wasserstoffatom oder die Methyl-, Ethyl-, Isopropyl-, Propyl-, tert Butyl-, Butyl-, Pentyl-, Hexyl-, Heptyl- oder Octylgruppe.

Besonders bevorzugt sind Verbindungen der allgemeinen Formel I, in der R⁴ ein Pyridinylring oder ein Phenyl der allgemeinen Formel II ist, in der
R⁵ Wasserstoff
R⁶ Wasserstoff oder Chlor bedeutet,
R⁷ Wasserstoff oder die Methoxygruppe ist,
R¹ ein Wasserstoffatom oder die Methylgruppe darstellt und
R² die Methyl-, Ethyl- oder Isopropylgruppe bedeutet, oder
R¹ und R² zusammen mit dem C-Atom, an das sie gebunden sind, einen Spirocyclopentylring darstellen,
R₃ das Wasserstoffatom, die Methyl-, Ethyl-, Propyl-, Isopropyl- oder Butylgruppe bedeutet.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Staerinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch 1 mal pro Tag 1-2 Tabletten mit 20-700 mg Wirkstoff gegeben werden müssen. Der Wirkstoff kann auch durch Injektion 1-8 mal pro Tag bzw. durch Dauerinfusion gegeben werden, wobei Mengen von 10-1000 mg pro Tag normalerweise ausreichen.

Zur Überführung der Verbindungen der allgemeinen Formel I bzw. deren tautomeren Formen in ihre pharmakologisch unbedenklichen Salze, setzt man diese vorzugsweise in einem organischen Lösungsmittel mit der äquivalenten Menge einer anorganischen oder organischen Säure, z.B. Salzsäure, Bromwasserstoffsäure, Phosphorsäure, Schwefelsäure, Essigsäure, Citronensäure, Weinsäure, Maleinsäure, Fumarsäure, Benzoesäure oder Cyclohexylsulfaminsäure um.

Die Imidazole der allgemeinen Formel I können nach an sich bekannten Verfahren hergestellt werden, wie sie in E.S. Schipper, A.R. Day, Hetercyclic Compounds (R.C. Elderfield, Editor), Band 5, S. 194 ff, Wiley, New York, 1957, beschrieben sind.

Besonders vorteilhaft ist die Synthese aus Amidinen und Halogenacylverbindungen. Setzt man die Amidine der allgemeinen Formel IV, in der R⁴ die oben angegebenen Bedeutungen hat, mit den Halogenacylverbindungen der allgemeinen Formel V, in der R¹, R², R³ und n die oben angegebenen Bedeutungen haben und Hal ein Halogenatom, vorzugsweise Chlor oder Brom bedeutet, um, so entstehen die Imidazole der allgemeinen Formel I', in der R¹-R⁴ und n die angegebenen Bedeutungen haben und R⁴ in 2-Stellung und der Bicyclus in 4-Stellung (oder 5-Stellung) am Imidazol steht.

Durch Umsetzung der Amidine der allgemeinen Formel VII mit den Halogenacylverbindungen der allgemeinen Formel VI entstehen die Imidazole der allgemeinen Formel I'', in der der Rest R⁴ in 4-Stellung (oder 5-Stellung) und der Bicyclus in 2-Stellung am Imidazolring steht. In den allgemeinen Formeln VI, VII und I'' haben R₁-R₄, n und Hal die oben angegebenen Bedeutungen. 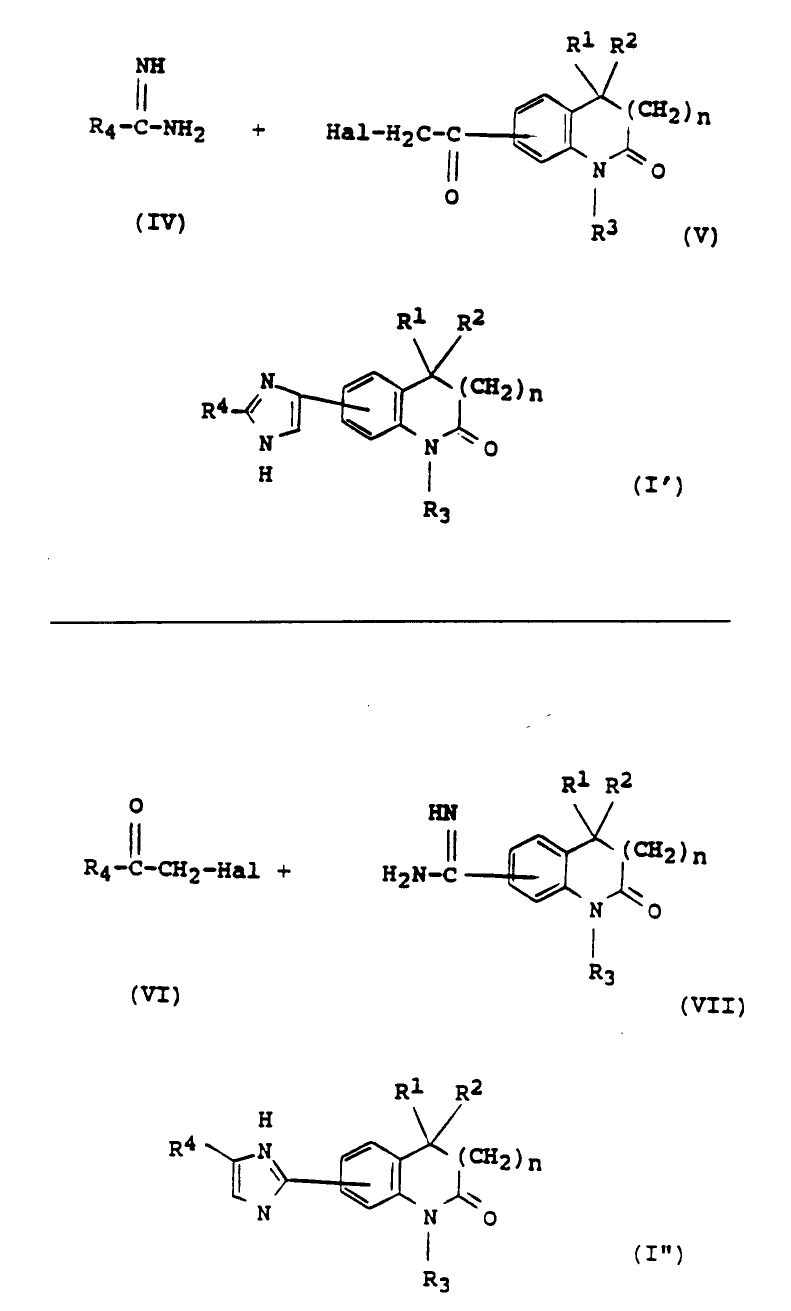
Die Umsetzung der Amidine IV, VII mit den Halogenacylverbindungen V, VI erfolgt in geeigneten organischen Lösungsmitteln, z. B. in Alkoholen wie Methanol, Ethanol oder Isopropanol, in Ethern wie Diethylether, Methyl-tert. Butylether oder Tetrahydrofuran oder einem anderen inerten Lösungsmittel wie Toluol, Essigester, Dimethylformamid oder Dimethylsulfoxid. Bevorzugt verwendet man einen Überschuß des Amidins. Verwendet man statt des freien Amidins dessen Hydrohalogenid, bevorzugt Hydrochlorid, Hydrobromid oder Hydroiodid, so wird dem Reaktionsmedium portionsweise ein Äquivalent einer Base, vorzugsweise Natriumhydroxid oder Natriumethylat, zugesetzt. Die Umsetzungen erfolgen bei Temperaturen zwischen O° C und dem Siedepunkt des Lösungsmittels.

Die Amidine der allgemeinen Formel IV sind käuflich oder literaturbekannt (H. Henecka, P. Kurtz in Methoden der Organischen Chemie (Houben, Weyl, Müller), Band VIII, S. 702ff, Thieme Verlag, Stuttgart 1952) oder können nach den darin beschriebenen Methoden hergestellt werden.

Halogenacylverbindungen der allgemeinen Formel V sind ebenfalls literaturbekannt (Europäische Patentanmeldung EP 303 418, Laboratoire Sobio S.A., 5.8.88) oder können nach den dort beschriebenen Methoden hergestellt werden. Sie können vorzugsweise hergestellt werden durch Umsetzung der am kondensierten Phenylring des Indol-2-on- bzw. Chinolin-2-on-systems unsubstituierten Derivate mit Halogenacetylhalogeniden, wie z. B. Chloracetylchlorid, in Gegenwart von Aluminiumchlorid. Halogenacylverbindungen der allgemeinen Formel VI sind käuflich oder literaturbekannt (vgl. Olah, "Friedel-Crafts and related reactions"; Vol. III, Interscience Publishers, New York, 1964; S. 20-22).

Die Verbindungen der allgemeinen Formel VII sind neu. Ihre Herstellung erfolgt nach den in H. Henecka, P. Kurtz in Methoden der Organischen Chemie (Houben-Weyl-Müller) Band VIII, S. 702 ff, Thieme Verlag, Stuttgart 1952 beschriebenen Methoden, vorzugsweise durch Umsetzung der Nitrile der allgemeinen Formel VIII 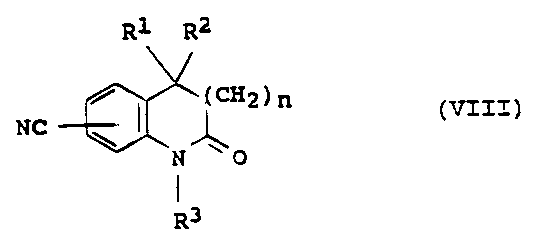
mit ethanolischer Salzsäure zu den Iminoethern der allgemeinen Formel IX, 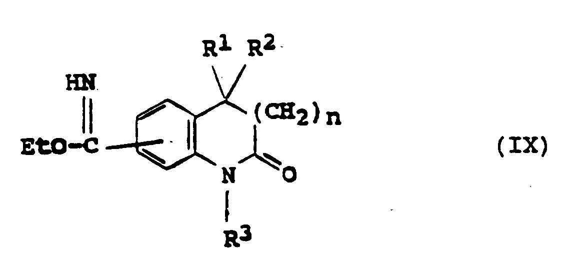
die vorzugsweise nicht isoliert werden, sondern mit ethanolischen Ammoniak zu den Verbindungen der allgemeinen Formel VII umgesetzt werden. In den allgemeinen Formeln VIII und IX haben R¹, R², R³ und n die oben angegebenen Bedeutungen. Die Nitrile der allgemeinen Formel VIII sind literaturbekannt (vgl. DE-A-3,818,830 bzw. EP-A-0,344,634).

Außer den in den Beispielen genannten Verbindungen der Formel I kommen beispielsweise auch die folgenden Verbindungen im Sinne der vorliegenden Erfindung in Frage:
1. 1,3-Dihydro-5-[2-phenyl-4(1H)-imidazolyl]-(2H)-indol-2-on
2. 1,3-Dihydro-3-methyl-5-[2-phenyl-4-(1H)-imidazolyl]-(2H)-indol-2-on
3. 1-3-Dihydro-3,3-diethyl-5-[2-phenyl-4(1H)-imidazolyl]-(2H)-indol-2-on
4. 1',3'-Dihydro-5'-[2-phenyl-4(1H)-imidazolyl]spiro[cyclopentan-1,3'-(2'H)indol]-2'-on
5. 1',3'-Dihydro-1'-methyl-5'-[2-phenyl-4(1H)-imidazolyl]-spiro[cyclopentan-1,3'-(2'H)indol]-2'-on
6. 1,3-Dihydro-3-isopropyl-5-[2-phenyl-4(1H)-imidazolyl]-(2H)-indol-2-on
7. 1,3-Dihydro-3-isopropyl-1-methyl-5-[2-phenyl-4(1H)-imidazolyl)-(2H)-indol-2-on

### Beispiel 1

1,3-Dihydro-3,3-dimethyl-1-ethyl-5-[2-phenyl-4(1H)-imidazolyl]-(2H)-indol-2-on
1,3-Diyhdro-3,3-dimethyl-1-ethyl-5-[(2-chlor-1-oxo)ethyl]-(2H)-indol-2-on (5.00 g, 18.8 mmol) und Benzamidin (3.40 g, 28.0 mmol) erhitzte man in Ethanol (300 mL) 4 h unter Rückfluß zum Sieden. Man entfernte das Lösungsmittel i. Vak. und trennte den Rückstand (11.1 g) chromatographisch an einer Umkehrphase (Säule: L 250 mm, Durchmesser 40 mm; Material Lichroprep RP 18 15-25 »m; Laufmittel: Methanol/Wasser/Ammoniumacetat = 75:25:0.1). Die dem Hauptpeak (UV-Detektor, 254 nm) entsprechenden Fraktionen werden eingedampft, der Rückstand (4.9 g) wurde in wenig Essigester aufgenommen, Ether zugegeben und das kristalline Produkt abgesaugt. Man erhielt 3.5 g (56 %) der Titelverbindung mit dem Fp. 190-194°C.

Die Vorstufe stellte man wie folgt her:
Zu 1,3-Dihydro-3,3-dimethyl-1-ethyl-(2H)-indol-2-on (30 g, 158 mmol) und Chloracetylchlorid (50 ml, 628 mmol) in Dichlormethan (250 ml) gab man unter Eiskühlung portionsweise Aluminiumchlorid (100 g, 749 mmol).

Man erhitzte anschließend 2 h unter Rückfluß zum Sieden, kühlte ab, goß vorsichtig auf Eis (ca. 2 l) und extrahierte zweimal mit Dichlormethan. Man trocknete die organische Phase über Natriumsulfat, filtrierte und entfernte das Lösungsmittel i.Vak. Man kristallisierte den Rückstand aus Ethanol um und erhielt 31 g (74 %) 1,3-Dihydro-3,3-dimethyl-1-ethyl-5-[(2-chlor-1-oxo)ethyl]-(2H)-indol-2-on mit dem Fp. 143-145°C.

### Beispiel 2

1,3-Dihydro-3,3-dimethyl-1-ethyl-5-[2-(4-chlorphenyl)-(1H)-4-imidazolyl]-(2H)-indol-2-on mit dem Fp. 169-175°C erhielt man in 64 % Ausbeute analog dem Beispiel 1 durch Umsetzung mit 4-Chlorbenzamidin.

### Beispiel 3

1,3-Dihydro-3,3-dimethyl-1-ethyl-5-[2-(4-methoxyphenyl)-4(1H)-imidazolyl]-(2H)-indol-2-on mit dem Fp. 128-133°C erhielt man in 47 % Ausbeute analog dem Beispiel 1 durch Umsetzung mit 4-Methoxybenzamidin.

### Beispiel 4

1,3-Dihydro-3,3-dimethyl-1-ethyl-5-[2-(3-chlorphenyl)-4(1H)-imidazolyl]-(2H)-indol-2-on mit dem Fp. 196-197°C erhielt man in 35 % Ausbeute analog dem Beispiel 1 durch Umsetzung mit 3-Chlorbenzamidin.

### Beispiel 5

1,3-Dihydro-3,3-dimethyl-1-ethyl-5-[2-(4-fluorphenyl)-4(1H)-imidazolyl]-(2H)-indol-2-on mit dem Fp. 162-165°C erhielt man in 40 % Ausbeute analog dem Beispiel 1 durch Umsetzung mit 4-Fluorbenzamidin.

### Beispiel 6

1,3-Dihydro-3,3-dimethyl-5-[2-(4-chlorphenyl)-4(1H)-imidazolyl]-(2H)-indol-2-on mit dem Fp. 137°C (Zers.) erhielt man in 20 % Ausbeute durch Umsetzung von 1,3-Dihydro-3,3-dimethyl-5-[(2-chlor-1-oxo)ethyl]-(2H)-indol-2-on (beschrieben in der Europäischen Patentanmeldung EP 303 418, 5.8.88, Laboratoire Sobio S.A.) mit 4-Chlorbenzamidin.

### Beispiel 7

1,3-Dihydro-3,3-dimethyl-5-[2-(4-fluorphenyl)-4(1H)-imidazolyl]-(2H)-indol-2-on mit dem Fp. 167-170°C erhielt man in 33 % Ausbeute analog dem Beispiel 6 durch Umsetzung mit 4-Fluorbenzamidin.

### Beispiel 8

1,3-Dihydro-3,3-dimethyl-5-[2-(3-chlorphenyl)-4(1H)-imidazolyl]-(2H)-indol-2-on mit dem Fp. 259-262°C (Zers.) erhielt man in 38 % Ausbeute analog dem Beispiel 6 durch Umsetzung mit 3-Chlorbenzamidin.

### Beispiel 9

1,3-Dihydro-3,3-dimethyl-5-[2-phenyl-4(1H)-imidazolyl]-(2H)-indol-2-on mit dem Fp. 267-277°C (Zers.) erhielt man in 38 % Ausbeute analog dem Beispiel 6 durch Umsetzung mit Benzamidin.

### Beispiel 10

3,4-Dihydro-4,4-dimethyl-1-ethyl-6-[2-phenyl-4-(1H)-imidazolyl]-2-(1H)chinolinon
Eine Lösung von Benzamidin-Hydrochlorid (1.6 g, 10 mmol), Natriumethylat (0.7 g, 10 mmol) und 3,4-Dihydro-4,4-dimethyl-1-ethyl-6-[(2-chlor-1-oxo)ethyl]-2(1H)-chinolinon (2.8 g, 10 mmol) in Ethanol (50 ml) erhitzte man 4 h unter Rückfluß zum Sieden. Nach 2 h und 3 h gab man nochmals Natriumethylat zu (0.4 g bzw. 0.3 g). Man filtrierte unlösliche Bestandteile, engte das Filtrat i.Vak. zur Trockene ein und reinigte den Rückstand chromatographisch an einer Umkehrphase (Bedingungen wie in Beispiel 1). Die entsprechenden Fraktionen engte man i.Vak. zur Trockene ein, digerierte das zurückbleibende gelbe Öl (1.2 g) mit Ether und saugte den kristallinen Niederschlag ab. Man erhielt 0.7 g (20 % ) der Titelverbindung mit dem Fp. 242-245°C.

Die Halogenacylverbindung 3,4-Dihydro-4,4-dimethyl-1-ethyl-6-[(2-chlor-1-oxo)ethyl]-2(1H)-chinolinon wurde in Analogie zu der in Beispiel 1 beschriebenen Methode hergestellt.

### Beispiel 11

1,3-Dihydro-3,3-dimethyl-6-[4-phenyl-2(1H)-imidazolyl]-(2H)-indol-2-on
2,3-Dihydro-3,3-dimethyl-2-oxo-(1H)-indol-6-carbox-imidamidhydrochlorid (6.1 g, 25 mmol) und Chloracetophenon (3.1 g, 20 mmol) setzte man in Ethanol (50 ml) in Gegenwart von Natriumhydroxid (30 mmol) wie in Beispiel 1 beschrieben miteinander um. Man erhielt 0.8 g (13 %) der Titelverbindung mit dem Fp. 242-249°C (Zers.).

Die Vorstufe stellte man wie folgt her:
2,3-Dihydro-3,3-dimethyl-2-oxo-(1H)-indol-6-carbonitril (20 g, 107 mmol; Herstellung beschrieben in DE-A-3,818,830 suspendierte man in Ethanol (500 ml) und leitete unter Kühlung bis zur sättigung Salzsäure ein. Man rührte 16 h bei Raumtemperatur, entfernte das Lösungsmittel i. Vak., gab Ethanol zu (500 ml), leitete Ammoniak ein und rührte 16 h bei Raumtemperatur. Man entfernte das Lösungsmittel i. Vak., digerierte den Rückstand mit Wasser, gab 2 N wäßrige Ammoniaklösung bis pH = 9-10 zu, filtrierte, wusch mit wenig Wasser und erhielt 19.3 g 2,3-Dihydro-3,3-dimethyl-2-oxo-(1H)-indol-6-carboximidamid.

### Beispiel 12

1,3-Dihydro-3,3-dimethyl-5-[2-(4-methoxyphenyl-4(1H)-imidazolyl]-(2H)-indol-2-on mit dem Fp. 263-266°C erhielt man in 10 % Ausbeute analog dem Beispiel 6 durch Umsetzung mit 4-Methoxybenzamidin.

### Beispiel 13

1,3-Dihydro-3,3-dimethyl-5-[2-(4-pyridinyl)-4(1H)-imidazolyl]-(2H)-indol-2-on mit dem Fp. >300°C erhielt man in 13 % Ausbeute analog dem Beispiel 6 durch Umsetzung mit 4-Pyridinylamidin.

### Beispiel 14

3,4-Dihydro-4,4-dimethyl-6-[2-phenyl-4(1H)-imidazolyl]-2(1H)-chinolinon mit dem Fp. 236-238°C erhielt man in 40 % Ausbeute analog dem Beispiel 10 durch Umsetzung von Benzamidin-Hydrochlorid mit 3,4-Dihydro-4,4-dimethyl-6-[(2-chlor-1-oxo)ethyl]-2(1H)-chinolinon, das man wie folgt herstellte:
Zu wasserfreiem Aluminiumchlorid (13.3 g, 140 mmol) tropfte man unter Eiskühlung trockenes Dimethylformamid (2.3 ml) und rührte die zähe Masse 30 min bei 70°C unter Feuchtigkeitsausschluß. Man ließ auf Raumtemperatur abkühlen, gab portionsweise 3,4-Dihydro-4 4-dimethyl-2(1H)-chinolinon (1.75 g, 140 mmol) und dann Chloracetylchlorid (0.8 ml, 10 mmol) zu und rührte 1 h bei Raumtemperatur. Man goß auf Eis, filtrierte und erhielt 3,4-Dihydro-4,4-dimethyl-6-[(2-chlor-1-oxo)-ethyl]-2(1H)-chinolinon (2 g, 80 %) mit dem Fp. 186-188°C.

### Beispiel 15

1,3-Dihydro-3,3-dimethyl-1-ethyl-5-[4-phenyl-2(1H)-imidazolyl]-(2H)-indol-2-on mit dem Fp. 195-197°C erhielt man in 26 % Ausbeute analog dem Beispiel 1 durch Umsetzung von (2-Chlor-1-oxo)ethylbenzol mit 1,3-Dihydro-3,3-dimethyl-1-ethyl-2-oxo-(2H)-5-indolcarboximidamid, das man wie folgt herstellte:
Zu 1,3-Dihydro-3,3-dimethyl-1-ethyl-(2H)-indol-2-on (38 g, 0.2 mol) in 80 % Schwefelsäure (300 ml) tropfte man bei 10-20°C ein Gemisch aus 80 % Schwefelsäure (80 ml) und 96 % Salpetersäure (10 ml). Man rührte noch 30 min bei Raumtemperatur, goß auf Eis (800 g), saugte ab, wusch mit Wasser, kristallisierte aus Ethanol um und erhielt 44 g (93 %) 2,3-Dihydro-3,3-dimethyl-1-ethyl-5-nitro-(1H)-indol-2-on mit dem Fp. 142-143°C. Diese Verbindungen (36 g, 153 mmol) hydrierte man in Ethanol (1 l) in Gegenwart von 2 g 10 % Pd auf C bis 11 l Wasserstoff aufgenommen waren. Man filtrierte, entfernte das Lösungsmittel i. Vak. und trug den Rückstand (31 g) in 2 N Salzsäure (300 ml) ein. Man kühlte auf 5°C und tropfte rasch eine Lösung von Natriumnitrit (12.5 g, 180 mmol) in Wasser (40 ml) zu. Man rührte 15 min bei 5°C und gab 2.5 g Harnstoff zu. Nach weiteren 15 min tropfte man diese Lösung zu einer Lösung von Natriumcyanid (29.5 g, 600 mmol), Kupfer(I)-cyanid (18.5 g, 210 mmol) und Natriumcarbonat (40.0 g, 380 mmol) in Wasser (1 l), das mit Toluol (300 ml) überschichtet war. Man rührte 16 h bei Raumtemperatur, trennte die organische Phase ab, schüttelte die wäßrige Phase mit Toluol aus und entfernte das Lösungsmittel der vereinigten Phasen i.Vak. Den Rückstand filtrierte man mit Toluol/Essigester (9:1, v/v) über Kieselgel (600 ml). Man engte das Filtrat i.Vak. ein und reinigte den Rückstand säulenchromatographisch (Säule wie Beispiel 1, Laufmittel: Wasser:Methanol:Ammoniumacetat = 20:80:0.1). Man erhielt 15.5 g (42 %) 1,3-Dihydro-3,3-dimethyl-1-ethyl-2-oxo-(2H)-5-indolcarbonitril mit dem Fp. 118-120°C.

Diese Verbindung (10 g, 46.7 mmol) löste man in trockenem Ethanol (80 ml) und leitete unter Kühlung bei 25°C bis zur Sättigung trockenen Chlorwasserstoff ein. Man ließ 24 h bei Raumtemperatur stehen und entfernte dann das Lösungsmittel i.Vak.. Den Rückstand löste man erneut in trockenem Ethanol (80 ml) und leitete bis zur Sättigung trockenen Ammoniak ein. Man rührte 24 h bei Raumtemperatur, entfernte das Lösungsmittel i.Vak. und setzte den Rückstand (1,3-Dihydro-3,3-dimethyl-1-ethyl-2-oxo-(2H)-indolcarboximidamid) gleich in der oben beschriebenen Reaktion ein.

### Beispiel 16

1,3-Dihydro-1,3,3-trimethyl-5-[4-phenyl-2(1H)-imidazolyl]-(2H)-indol-2-on, das bei 191-194°C schmilzt, wieder erstarrt und bei 225-227°C erneut schmilzt, erhielt man in 27 % Ausbeute analog dem Beispiel 1 durch Umsetzung von -Chloracetophenon mit 1,3-Dihydro-3,3-dimethyl-1-methyl-2-oxo-(2H)-5-indolcarboximidamid, das man wie folgt herstellte:
Unter Kühlung mit Eiswasser tropfte man Phosphoroxichlorid (34 ml, 375 mmol) zu N-Methylformanilid (41 ml, 370 mmol), dann gab man bei Raumtemperatur 2,3-Dihydro-1,3,3-trimethyl-2(1H)-indolon (44 g, 250 mmol) zu. Man rührte 24 h bei 50°C, gab Eiswasser zu, säuerte mit konz. Salzsäure an, extrahierte mit Teoluol/Essigester 10:1. Die organische Phase trocknete man über Natriumsulfat, filtrierte und entfernte das Lösungsmittel i.Vak.. Die Hälfte (26 g) des Rückstandes löste man in Ethanol (100 ml) und gab Hydroxylamin-Hydrochlorid (10.5 g) zu. Dann tropfte man 10 N Natronlauge (12.5 ml) zu. Dabei begann bereits das gewünschte Oxim auszukristallisieren. Man verdünnte in Portionen während 1 h mit insgesamt 150 ml Waser. Man saugte ab, wusch mit Wasser und erhielt 2,3-Dihydro-1,3,3-trimethyl-2-oxo-(1H)-5-indolcarboxaldelydoxim (24 g, 86 %) mit dem Fp. 134-136°C.

Diese Verbindung (23.6 g, 11.6 mmol) und Pyridin (0.2 ml) erhitzte man in Essigsäureanhydrid (40 ml) 1.5 h unter Rückfluß zum Sieden. Nach dem Abkühlen gab man Eis und Wasser zu, saugte das kristalline Produkt ab, wusch mit Wasser und erhielt 2,3-Dihydro-1,3,3-trimethyl-2-oxo-(1H)-5-indolcarbonitril (19.1 g, 88 %) mit dem Fp. 178-182°C.

Aus dieser Verbindung bereitete man analog Beispiel 15 2,3-Dihydro-1,3,3-trimethyl-2-oxo-(1H)-5-indolcarboximidamid, das man ohne weitere Reinigung in der oben beschriebenen Reaktion einsetzte.

### Beispiel 17

1,3-Dihydro-1,3,3-trimethyl-5-[2-phenyl-4(1H)-imidazolyl]-(2H)-indol-2-on, mit dem Fp. 259-260°C erhielt man in 21 % Ausbeute analog dem Beispiel 1 durch Umsetzung von Benzamidin mit 5-(2-Chloracetyl)-1,3-dihydro-1,3,3-trimethyl-2H-indol-2-on, das man wie folgt herstellte:
Zu einer Lösung von Chloracetylchlorid (50 ml) und 1,3-Dihydro-1,3,3-trimethyl-2H-indol-2-on (29 g, 166 mmol) in Dichlormethan gab man portionsweise Aluminiumchlorid (100 g). Man erhitzte 2 h unter Rückfluß zum Sieden, goß auf Eis und extrahierte mit Dichlormethan. Man entfernte das organische Lösungsmittel i. Vak., kristallisierte aus Ethanol um und erhielt 28 g (67 %) 5-(2-Chloracetyl)-1,3-dihydro-1,3,3-trimethyl-2H-indol-2-on mit dem Fp. 185-187°C.

### Beispiel 18

1,3-Dihydro-1,3,3-trimethyl-6-[2-phenyl-4(1H)-imidazoyl]-(2H)-indol-2-on mit dem Fp. 221° C erhielt man in 37 % Ausbeute analog dem Beispiel 1 durch Umsetzung von Benzamidin mit 6-Bromacetyl-1,3-dihydro-1,3,3-trimethyl-2H-indol-2-on, das man wie folgt herstellte:
a) 2,3-Dihydro-1,3,3-trimethyl-2-oxo-(1H)-indol-6-carbonitril
   Zu einer Lösung von 2,3-Dihydro-3,3-dimethyl-2-oxo-(1H)-indol-6-carboxymid (28 g, 154 mmol; Herstellung beschrieben in DE-A-3,818,830 in Tetrahydrofuran (55 ml) und Toluol (70 ml) tropfte man konzentrierte Natronlauge (56 ml). Dabei stieg die Temperatur von 20° C auf 45° C an. Zur Suspension gab man Benzyltributylammoniumbromid (1.1 g) und tropfte Jodmethan (14.1 ml) zu. Man rührte 2 h bei 45° C nach und goß auf Eiswasser (500 ml). Den Niederschlag saugte man ab und wusch mit Wasser. Man erhielt so 22.3 g der Titelverbindung mit dem Fp. 143-145° C. Aus der Mutterlauge wurden nochmals 7.1 g gewonnen (Ausbeute 98 %).
b) 2,3-Dihydro-1,3,3-trimethyl-2-oxo-(1H)-indol-6-carbonsäure
   2,3-Dihydro-1,3,3-trimethyl-2-oxo-(1H)-indol-6-carboxamid (19 g, 95.4 mmol) wurden in 2 N Natronlauge (700 ml) 2 h bei 80° C gerührt. Man säuerte mit 6 N Salzsäure an, saugte den Niederschlag ab und wusch mit Wasser. Man erhielt 20.1 g (97 %) der Titelverbindung mit dem Fp. 253-255° C.
c) 2,3-Dihydro-1,3,3-trimethyl-2-oxo-(1H)-indol-6-carbonsäurechlorid
   Die oben beschriebene Carbonsäure (7.7 g) erhitzte man in Thionylchlorid (100 ml), das einen Tropfen DMF enthielt, 1 h unter Rückfluß zum Sieden. Dann dampfte man i.Vak. vollständig ein. Der Rückstand wurde ohne weitere Reinigung in der nächsten Stufe eingesetzt.
d) 6-Acetyl-1,3-dihydro-1,3,3-trimethyl-2H-indol-2-on
   Man überschichtete Magnesiumspäne (2 g, 84.1 mmol) mit Ether, gab 4 Tropfen Dibromethan und nach dem Anspringen der Reaktion Ethanol zu (3 ml). Man erhielt das Lösungsmittel mit dem Wasserbad am Sieden und tropfte unter starkem Rühren eine Lösung aus Malonsäurediethylester (12.8 ml, 84.1 mmol) in Ethanol (8 ml) und Ether (10 ml) zu. Danach versetzte man mit Ether (20 ml) und rührte, bis sich das Magnesium gelöst hatte. Zu diesem Zeitpunkt entstand in einigen Fällen ein Niederschlag. Man gab dann weiter Ether zu, bis eine gut rührbare Suspension entstand. Unter Sieden tropfte man dazu eine Suspension von 2,3-Dihydro-1,3,3-trimethyl-2-oxo-(1H)-indol-6-carbonsäurechlorid (20 g, 84.1 mmol) in Ether (100 ml). Man hielt noch 30 min am Sieden, kühlte ab und säuberte mit 2 N Salzsäure an. Von der nunmehr klaren Lösung trennte man die organische Phase ab, extrahierte die wässrige Phase mit Ether, trocknete und entfernte das Lösungsmittel i.Vak.. Das zurückbleibende Öl versetzte man mit Wasser (33 ml), Eisessig (42 mol), konzentrierter Schwefelsäure (9 ml) und hielt die Lösung 5 h bei 90° C. Man versetzte nach dem Abkühlen mit Wasser und saugte das ausgefallene Produkt ab. Man erhielt 8.3 g (47 %) der Titelverbindung mit dem Fp. 128-130° C.
e) 6-Bromacetyl-1,3-dihydro-1,3,3-trimethyl-2H-indol-2-on
   Zu 6-Acetyl-1,3-dihydro-1,3,3-trimethyl-2H-indol-2-on (6.1 g) in Eisessig (60 ml) gab man konzentrierte Salzsäure (0.5 ml) und tropfte bei etwa 10° C innerhalb von 20 min Brom (0.8 ml) in Eisessig (19 ml) zu. Man rührte 3 h bei Raumtemperatur, extrahierte dreimal mit Essigester (je 300 ml), trocknete die organische Phase, entfernte das Lösungsmittel, rieb das zurückbleibende Öl mit Ether an und erhielt 3.1 g (42 %) der Titelverbindung mit dem Fp. 104° C.

### Beispiel 19

3,4-Dihydro-4,4-dimethyl-7-[4-phenyl-2(1H)-imidazolyl]-2(1H)-chinolinon mit dem Fp. 270-271° C erhielt man in 24 % Ausbeute durch Umsetzung von Chloracetophenon mit 3,4-Dihydro-4,4-dimethyl-2-oxo-1H-7-chinolincarboximidamid-Hydrochlorid analog dem Beispiel 11 und anschließender Umkristallisation aus Essigester.

### Beispiel 20

1,3-Dihydro-1,3,3-trimethyl-6-[4-phenyl-2(1H)-imidazolyl]-(2H)-indol-2-on erhält man als harten Schaum durch Umsetzung von Chloracetophenon mit 2,3-Dihydro-1,3,3-trimethyl-2-oxo-(1H)-indol-6-carboximidamid-hydrochlorid in 33 % Ausbeute analog dem Beispiel 11.

### Beispiel 21

1,3-Dihydro-1-ethyl-3,3-dimethyl-6-[4-phenyl-2(1H)-imidazolyl]-(2H)-indol-2-on erhielt man als harten Schaum durch Umsetzung von Chloracetophenon mit 2,3-Dihydro-3,3-dimethyl-1-ethyl-2-oxo-(1H)-indol-6-carboximidamid-hydrochlorid in 28 % Ausbeute analog dem Beispiel 11.

### Beispiel 22

### Hämorheologische Untersuchungen

Die Bestimmung der Erythrozyten-Aggregation als Parameter für die Hämorheologie erfolgte mit dem Mini-Erythrozyten-Aggregometer der Fa. Myrenne, Roetgen¹). Als Maß gibt dieses Gerät einen dimensionslosen Index an, der mit steigender Aggregationstendenz zunimmt.

Die Untersuchungen wurden mit dem Humanblut gesunder Spender durchgeführt. Das auf einen Haematokrit von 45 % eingestellte Blut wurde mit der Kontrollösung oder den Substanzlösungen inkubiert. Anschließend wurde die Erythrozyten-Aggregation gemessen. Jede Substanz wurde bei einer Konzentration von 10⁻⁵ M untersucht. Pro Substanz wurden zwei Versuche mit dem Blut verschiedener Spender durchgeführt. Es wurde die Differenz der Aggregationsindices zwischen dem Ausgangswert der Kontrollösung und den Werten der Substanzlösung errechnet. Diese Werte sind in der folgenden Tabelle aufgelistet.

Venoruton^{R}, ein Gemisch aus verschiedenen O-(β-Hydroxyethyl)rutosiden, soll die Tendenz der Erythrozyten-Aggregation hemmen²). In der obigen Versuchsanordnung bewirkt es bei einer vergleichbaren Konzentration von 1.7x10⁻⁵ M eine Änderung des Aggregationsindex lediglich um -0.4 Einheiten. Selbst bei einer Konzentration von 1.7x10⁻³ M beträgt die Änderung nur -3.9+/-0.9. Im Vergleich zu Venoruton^{R} hemmen die genannten Substanzen die Erythrozyten-Aggregation deutlich stärker.

### Literatur:

1) Kiesewetter, H. et al., Das mini-Erythrozyten-Aggregometer: Ein neues Gerät zur schnellen Quantifizierung des Ausmaßes der Erythrozyten-Aggregation. Biomed. Technik. 27 (1982), Heft 9, S. 209-213.
2) Schmid-Schönbein, H. et al., Effext of O-(β-Hydroxyethyl)rutosides on the Microrheology of Human Blood under Defined Flow Conditions. VASA 4 (1975) 262-270.

**Tabelle**

| Differenz der Erythrozytenaggregationsindices (E₂-E₁) | |
|---|---|
| Beispiel | E2-E1 |
| 5 | -8.7 |
| 6 | -8.2 |
| 14 | -7.2 |
| 17 | -8.6 |

## Patentansprüche

1. Bicyclo-Imidazole der Formel I 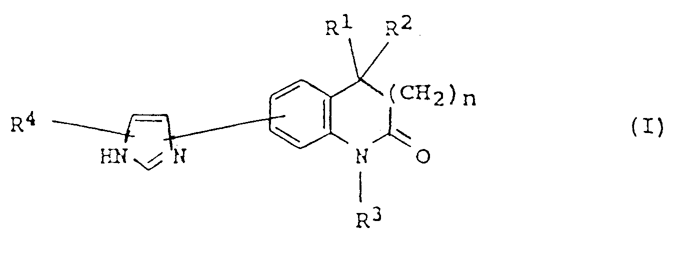 in der
R¹ ein Wasserstoffatom, eine C₁-C₆-Alkyl-, C₂-C₆-Alkenyl- oder eine C₃-C₇-Cycloalkylgruppe bedeutet,
R² eine C₁-C₆-Alkylgruppe bedeutet oder R¹ und R² zusammen mit dem Kohlenstoffatom, an das sie gebunden sind, einen C₃-C₇-Spirocyclus bilden,
n gleich 0 oder 1 sein kann,
R³ ein Wasserstoffatom oder eine C₁-C₈-Alkylgruppe bedeutet,
R⁴ einen in 2-oder-4-Stellung des Imidazols befindlichen Pyridinyl- oder Phenylring der Formel II bedeutet, 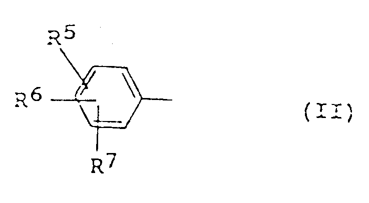
in dem
R⁵, R⁶, R⁷ gleich oder verschieden sein können und jeweils Wasserstoff, Halogen oder C₁-C₇-Alkoxy bedeuten,
wobei im Fall n=0 der Imidazolring in 4-, 5-, 6- oder 7-Stellung mit dem 2,3-Dihydroindolin-2-on oder im Fall n=1 in 5-, 6-, 7-, 8-Stellung mit dem 1, 2,3,4-Tetrahydrochinolin-2-on verknüpft sein kann, deren Tautomere, optisch aktive Formen oder deren physiologisch verträgliche Salze.

2. Bicyclo-Imidazole gemäß Anspruch 1, wobei
R⁵ Wasserstoff oder ein Halogenatom,
R⁶ Wasserstoff oder ein Halogenatom und
R⁷ Wasserstoff oder die Methoxygruppe bedeuten.

3. Bicyclo-Imidazole gemäß Anspruch 1 oder 2, wobei
R⁴ ein Pyridinylring oder ein Phenyl der allgemeinen Formel II ist, in der
R⁵ Wasserstoff,
R⁶ Wasserstoff oder Chlor bedeutet,
R⁷ Wasserstoff oder die Methoxygruppe ist
R¹ ein Wasserstoffatom oder die Methylgruppe darstellt und
R² die Methyl-, Ethyl- oder Isopropylgruppe bedeutet, oder
R¹ und R² zusammen mit dem C-Atom, an das sie gebunden sind, einen Spirocyclopentylring darstellen,
R₃ das Wasserstoffatom, die Methyl-, Ethyl-, Propyl-, Isopropyl- oder Butylgruppe bedeutet.

4. Bicyclo-Imidazole gemäß einem der Ansprüche 1-3, dadurch gekennzeichnet, daß R⁵ den am Phenylring ortho-ständigen Substituenten darstellt und ein Wasserstoffatom bedeutet, R⁶ den meta-ständigen Substituenten darstellt und ein Wasserstoff- oder Halogenatom bedeutet, und R⁷ den para-ständigen Substituenten darstellt und ein Wasserstoff- oder Halogenatom oder eine C₁-C₆-Alkoxygruppe bedeutet.

5. Bicyclo-Imidazole gemäß einem der Ansprüche 1-4 ausgewählt aus der Gruppe der folgenden Verbindungen:
1,3-Dihydro-3,3-dimethyl-1-ethyl-5-[2-(4-fluorphenyl)-4(1H) - imidazolyl]-(2H)-indol-2-on,
1,3-Dihydro-3,3-dimethyl-5-[2-(4-chlorphenyl)-4(1H)-imidazolyl]-(2H)-indol-2-on,
1,3-Dihydro-3,3-dimethyl-5-[2-phenyl-4(1H)-imidazolyl]-(2H) - indol-2-on,
1,3-Dihydro-3,3-dimethyl-5-[2-(4-methoxyphenyl-4(1H)-imidazolyl]-(2H)-indol-2-on,
3,4-Dihydro-4,4-dimethyl-6-[2-phenyl-4(1H)-imidazolyl]-2(1H)-chinolinon,
1,3-Dihydro-3,3-dimethyl-1-ethyl-5-[4-phenyl-2(1H)-imidazolyl]-(2H)-indol-2-on oder
1,3-Dihydro-1,3,3-trimethyl-5-[2-phenyl-4(1H)-imidazolyl]-(2H)-indol-2-on.

6. Arzneimittel enthaltend mindestens eine Verbindung gemäß einem der Ansprüche 1-5 neben üblichen pharmakologischen Träger- oder Hilfsstoffe.

7. Verwendung von Verbindungen gemäß einem der Ansprüche 1-5 zur Herstellung von Arzneimitteln zur Behandlung von Krankheiten, bei denen eine Hemmung der Erythrozyten- oder Thrombozytenaggregation zweckmäßig ist.

8. Verfahren zur Herstellung von Bicyclo-Imidazolen gemäß den Ansprüchen 1-5, dadurch gekennzeichnet, daß man
a) eine Verbindung der Formel IV 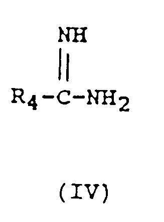 mit einer Verbindung der Formel V 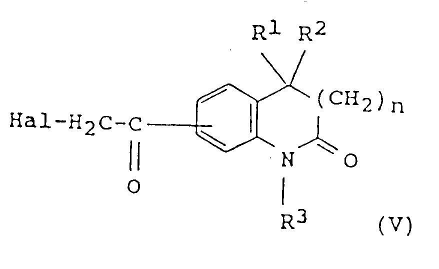
b) eine Verbindung der Formel VI 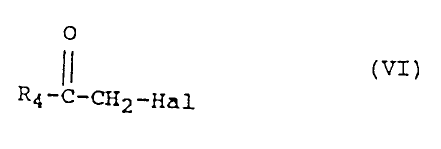 mit einer Verbindung der Formel VII 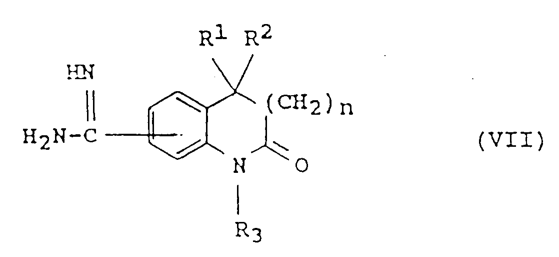
in einem inerten organischen Lösungsmittel bei geeigneten Temperaturen umsetzt, wobei R¹-R⁴ und n die in den Ansprüchen 1-4 angegebenen Bedeutungen haben und Hal für ein Halogenatom steht, und
gegebenenfalls anschließend die so erhaltenen Verbindungen der Formel I in ihre pharmakologisch verträglichen Salze mit anorganischen oder organischen Säuren überführt.

9. Verbindungen der Formel VII 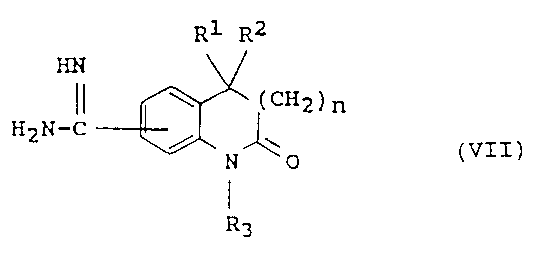 wobei R¹-R³ und n die in den Ansprüchen 1-3 angegebenen Bedeutungen haben.

## Claims

1. Bicycloimidazoles of the formula I 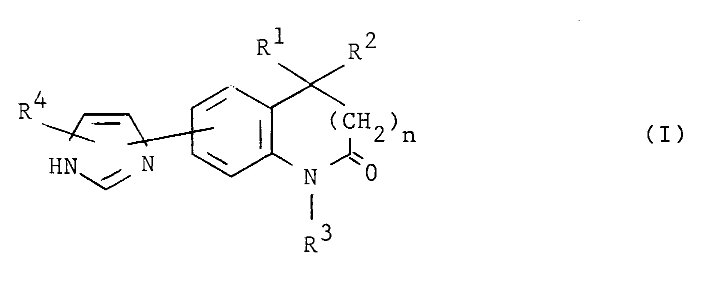 in which R¹ signifies a hydrogen atom, a C₁-C₆-alkyl, C₂-C₆-alkenyl or a C₃-C₇-cycloalkyl group, R² signifies a C₁-C₆-alkyl group or R¹ and R², together with the carbon atom to which they are bound, form a C₃-C₇-spirocycle, n can be equal to 0 or 1, R³ signifies a hydrogen atom or a C₁-C₈-alkyl group, R⁴ signifies a pyridinyl or phenyl ring of the formula II 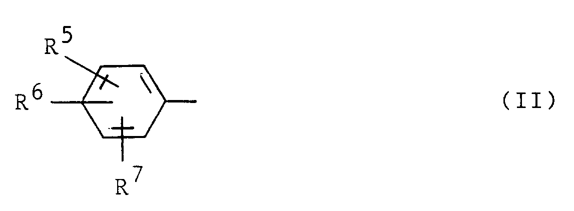 present in the 2- or 4-position of the imidazole, in which R⁵, R⁶, R⁷ can be the same or different and, in each case, signify hydrogen, halogen or C₁-C₇-alkoxy, whereby, in the case of n = 0, the imidazole ring can be attached in 4-, 5-, 6- or 7-position with the 2,3-dihydroindolin-2-one or, in the case of n = 1, in the 5-, 6-, 7-, 8-position with the 1,2,3,4-tetrahydroquinolin-2-one, their tautomers, optically-active forms or their physiologically compatible salts.

2. Bicycloimidazoles according to claim 1, whereby R⁵ signifies hydrogen or a halogen atom, R⁶ hydrogen or a halogen atom and R⁷ hydrogen or the methoxy group.

3. Bicycloimidazoles according to claim 1 or 2, whereby R⁴ is a pyridinyl ring or a phenyl of the general formula II, in which R⁵ signifies hydrogen, R⁶ hydrogen or chlorine, R⁷ is hydrogen or the methoxy group, R¹ represents a hydrogen atom or the methyl group and R² signifies the methyl, ethyl or isopropyl group or R¹ and R², together with the C-atom to which they are attached, represents a spirocyclopentyl ring, R³ signifies the hydrogen atom, the methyl, ethyl, propyl, isopropyl or butyl group.

4. Bicycloimidazoles according to one of claims 1 - 3, characterised in that R⁵ represents the substituents ortho-standing on the phenyl ring and signifies a hydrogen atom, R⁶ represents the meta-standing substituents and signifies a hydrogen or halogen atom and R⁷ represents the para-standing substituents and signifies a hydrogen or halogen atom or a C₁-C₆-alkoxy group.

5. Bicycloimidazoles according to one of claims 1 - 4, selected from the group of the following compounds:
1,3-dihydro-3,3-dimethyl-1-ethyl-5-[2-(4-fluorophenyl)-4(1H)-imidazolyl]-(2H)-indol-2-one,
1,3-dihydro-3,3-dimethyl-5-[2-(4-chlorophenyl)-4(1H)-imidazolyl]-(2H)-indol-2-one,
1,3-dihydro-3,3-dimethyl-5-[2-phenyl-4(1H)-imidazolyl]-(2H)-indol-2-one,
1,3-dihydro-3,3-dimethyl-5-[2-(4-methoxyphenyl-4(1H)-imidazolyl]-(2H)-indol-2-one,
3,4-dihydro-4,4-dimethyl-6-[2-phenyl-4(1H)-imidazolyl]-2(1H)-quinolinone,
1,3-dihydro-3,3-dimethyl-1-ethyl-5-[4-phenyl-2(1H)-imidazolyl]-(2H)-indol-2-one or
1,3-dihydro-1,3,3-trimethyl-5-[2-phenyl-4(1H)-imidazolyl]-indol-2-one.

6. Medicaments containing at least one compound according to one of claims 1 - 5, besides usual pharmacological carrier and adjuvant materials.

7. Use of compounds according to one of claims 1 - 5 for the preparation of medicaments for the treatment of diseases in which an inhibition of the erythrocyte or thrombocyte aggregation is expedient.

8. Process for the preparation of bicycloimidazoles according to claims 1 - 5, characterised in that one
a) reacts a compound of the formula IV 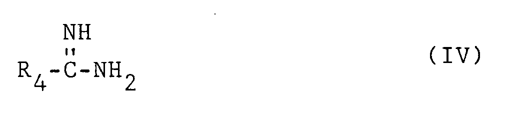 with a compound of the formula V 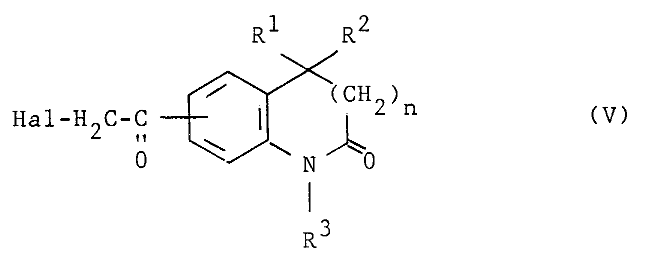
b) a compound of the formula VI 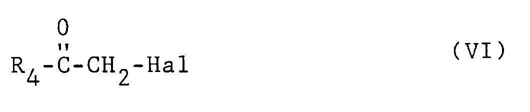 with a compound of the formula VII 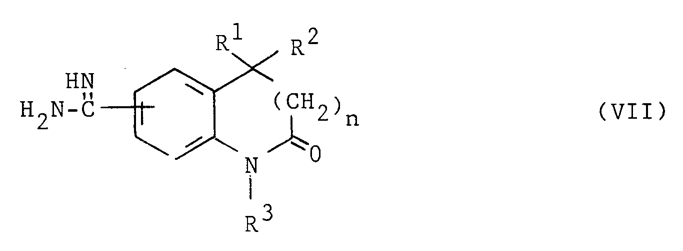
in an inert organic solvent at suitable temperatures, whereby R¹ - R⁴ and n have the meanings given in claims 1 - 4 and Hal stands for a halogen atom, and possibly subsequently converts the so-obtained compounds of the formula I into their pharmacologically compatible salts with inorganic or organic acids.

9. Compounds of the formula VII 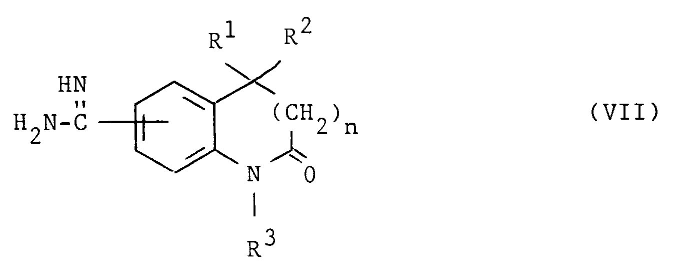 whereby R¹ - R³ and n have the meanings given in claims 1 - 3.

## Revendications

1. Bicyclo-imidazoles de formule I 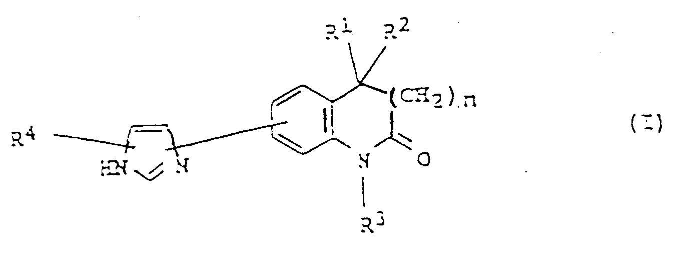 dans laquelle
R¹ représente un atome d'hydrogène, un groupe alkyle en C₁-C₆, alcényle en C₂-C₆ ou cycloalkyle en C₃-C₇,
R² représente un groupe alkyle en C₁-C₆, ou R¹ et R² forment, conjointement avec l'atome de carbone auquel ils sont liés, un spirocycle en C₃-C₇,
n peut prendre les valeurs de 0 ou 1,
R³ représente un atome d'hydrogène ou un groupe alkyle en C₁-C₈,
R⁴ représente un cycle pyridinyle ou phényle, se trouvant à la position 2 ou 4 de l'imidazole, répondant à la formule II 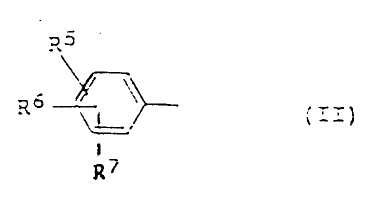
dans laquelle
R⁵, R⁶, R⁷ peuvent être identiques ou différents et représentent chacun un atome d'hydrogène d'halogène ou un groupe alcoxy en C₁-C₇,
étant entendu que dans le cas où n=0, le cycle imidazole peut être lié à la 2,3-dihydroindolin-2-one en position 4, 5, 6 ou 7, ou dans le cas où n=1, il peut être lié à la 1,2,3,4-tétrahydroquinoléin-2-one en position 5, 6, 7 ou 8,
leurs tautomères, leurs formes optiquement actives ou leurs sels physiologiquement acceptables.

2. Bicyclo-imidazoles selon la revendication 1, où
R⁵ représente un atome d'hydrogène ou d'halogène,
R⁶ représente un atome d'hydrogène ou d'halogène et
R⁷ représente un atome d'hydrogène ou un groupe méthoxy.

3. Bicyclo-imidazoles selon la revendication 1 ou 2, où
R⁴ représente un cycle pyridinyle ou un cycle phényle de formule générale II, dans laquelle
R⁵ représente un atome d'hydrogène,
R⁶ représente un atome d'hydrogène ou de chlore,
R⁷ représente un atome d'hydrogène ou un groupe méthoxy,
R¹ représente un atome d'hydrogène ou un groupe méthyle et
R² représente un groupe méthyle, éthyle ou isopropyle, ou
R¹ et R² forment, conjointement avec l'atome de C auquel ils sont liés, un spirocyclopentyle,
R³ représente un atome d'hydrogène, un groupe méthyle, éthyle, propyle, isopropyle ou butyle.

4. Bicyclo-imidazoles selon l'une quelconque des revendications 1-3, caractérisées en ce que R⁵ représente les substituants se trouvant en position ortho sur le cycle phényle et un atome d'hydrogène, R⁶ représente les substituants se trouvant en position méta et un atome d'hydrogène ou d'halogène et R⁷ représente les substituants se trouvant en position para et un atome d'hydrogène ou d'halogène ou un groupe alcoxy en C₁-C₆.

5. Bicyclo-imidazoles selon l'une quelconque des revendications 1-4, choisis dans le groupe comprenant les composés suivants :
1,3-dihydro-3,3-diméthyl-1-éthyl-5-[2-(4-fluorophényl)-4(1H)-imidazolyl]-(2H)-indol-2-one,
1,3-dihydro-3,3-diméthyl-5-[2-(4-chlorophényl)-4(1H)-imidazolyl]-(2H)-indol-2-one,
1,3-dihydro-3,3-diméthyl-5-[2-phényl-4(1H)-imidazolyl]-(2H)-indol-2-one,
1,3-dihydro-3,3-diméthyl-5-[2-(4-méthoxyphényl)-4(1H)-imidazolyl]-(2H)-indol-2-one,
3,4-dihydro-4,4-diméthyl-6-[2-phényl-4(1H)-imidazolyl]-2(1H)-quinoléinone,
1,3-dihydro-3,3-diméthyl-1-éthyl-5-[4-phényl-2(1H)-imidazolyl]-(2H)-indol-2-one,
ou
1,3-dihydro-1,3,3-triméthyl 5-[2-phényl-4(1H)-imidazolyl]-(2H)-indol-2-one.

6. Médicament contenant au moins un composé selon l'une quelconque des revendications 1 à 5, en plus de véhicules ou adjuvants pharmacologiques usuels.

7. Utilisation de composés selon l'une quelconque des revendications 1 à 5, pour la préparation de médicaments destinés au traitement de maladies pour laquelle une inhibition de l'aggrégation des érythrocytes ou des thrombocytes est recommandée.

8. Procédé de préparation de bicyclo-imidazoles selon l'une quelconque des revendications 1 à 5, caractérisé en ce que l'on fait réagir
a) un composé de formule IV 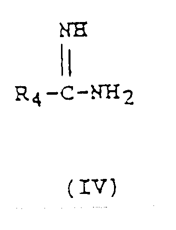 avec un composé de formule V 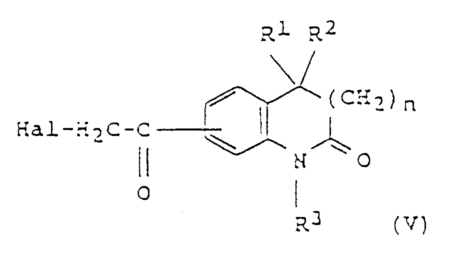
b) un composé de formule VI 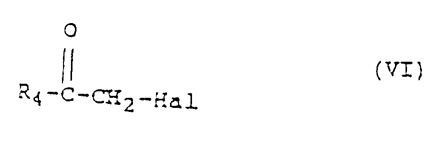 avec un composé de formule VII 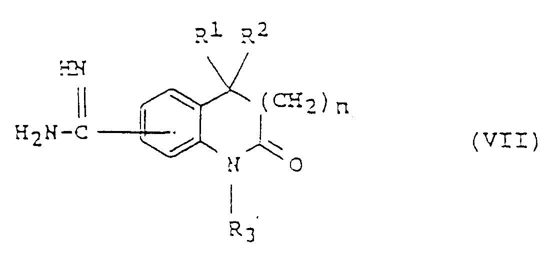
dans un solvant organique inerte à une température appropriée, R¹-R⁴ et n ayant les significations mentionnées dans les revendications 1 à 4 et Hal représentant un atome d'halogène, et
on transforme éventuellement les composés de formule I ainsi obtenus en leurs sels pharmacologiquement acceptables, formés avec des acides inorganiques ou organiques.

9. Composés de formule VII 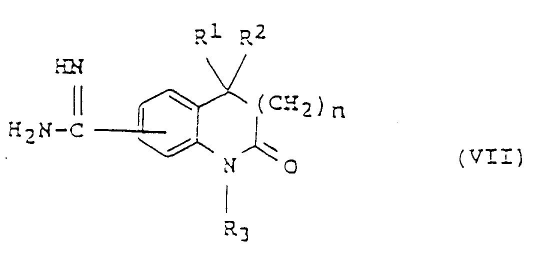 où R¹-R³ et n ont les significations mentionnées dans les revendications 1 à 3.
